# EUROPEAN PATENT APPLICATION

(11) **EP 1 416 418 A2**
(43) Date of publication of application: **06.05.2004**
(21) Application number: 03024268.9
(22) Date of filing: 23.10.2003
(51) Int. Cl.: G06F 19/00

(54) **Medical image terminal device, medical image radiographing system, display control method and program**

(30) Priority: 31.10.2002 JP 2002317226
(71) Applicant: KONICA MINOLTA HOLDINGS, INC., Tokyo 100-0005 (JP)
(72) Inventor: Moriyama, Naoto, Hachioji-shi Tokyo 192-8505 (JP)
(74) Representative: Gille Hrabal Struck Neidlein Prop Roos

(57) **Abstract**

A medical image terminal device capable of performing radiography efficiently and preventing medical radiographing errors by setting correspondence of a patient to radiographing order information accurately and easily. The medical image terminal device has: a receiving section to receive identification information of a patient to be radiographed and radiographing order information including information regarding radiography; a storage section to store the radiographing order information received; an input section to input the identification information of the patient in; and a display control section to obtain the radiographing order information stored in the storage section based on the identification information of the patient input and display the radiographing order information on a display section.

## Description

### BACKGROUND OF THE INVENTION

### Field of the Invention

The invention relates to a medical image terminal device in a medical image radiographing system, the medical image radiographing system, a display control method and a program.

### Description of Related Art

In a medical field, for example, a medical image radiographing system such as a CT (Computed Tomography), a CR (Computed Radiography), an MRI (Magnetic Resonance Imaging) or the like, has been used. In the medical image radiographing system, a medical image is obtained as digital image data by radiographing a patient as a subject.

Concretely, in a medical image radiographing apparatus, a medical image conversion panel wherein a photostimulable phosphor layer is formed on a support member, is used. The photostimulable phosphor sheet of the conversion panel absorbs radiations transmitting through the subject and forms a latent image by accumulating radiation energy corresponding to radiation transmittance through each part of the subject. Then, the phosphor layer is scanned by excitation light such as infrared rays or the like to stimulate the accumulated radiation energy into fluorescence, and the fluorescence is photoelectrically converted to obtain a medical image signal. Then, after getting an image process applied on, the obtained medical image is output to an output apparatus such as a CRT or the like to be visualized, or stored along with patient information in a filing apparatus such as a server or the like, to be used for medical service.

Such a medical image radiographing system is roughly classified into two system structures. One is a medical image radiographing system wherein an apparatus for both radiographing and reading an image and the photostimulable phosphor plate are arranged fixedly in a radiographing room to perform radiography. In the system, it is possible to radiograph and read the image simultaneously in the radiography room.

Another is a medical image radiographing system wherein radiography is performed at a patient's location with a portable radiographing apparatus for use of radiographing at the patient's location and a portable cassette incorporating a phosphor plate therein due to the reason that the patient has a broken bone or a cerebrobascular disease, or is under control in an ICU and therefore it is not possible to perform radiography in the radiography room. In the system, after the radiography is performed, the cassette is inserted into a reading apparatus for cassette use exclusively to read the radiographed image.

When radiography is performed in either of the two above-mentioned medical image radiographing systems, an operator confirms a patient to be radiographed in order to prevent a mix-up of patients. Generally, the operator directly calls to the patient. However, when radiography is performed with the portable radiographing apparatus at a hospital room of the patient, there is a case where the operator cannot communicate with the patient. In this case, the operator confirms the patient by checking patient identification information written in a name plate at the bedside, a wristband worn on an arm of the patient or the like (for example, see Japanese Patent Application Publication (Unexamined) Nos. Tokukai-hei 10-49604, Tokukai 2001-175776 and Tokukai 2002-123607).

Then, at the time of radiography, after the operator recognizes radiographing order information on an order sheet corresponding to the patient confirmed as the one to be radiographed to check a radiographing condition, radiographing counts and the like, the operator performs radiography. Here, on the order sheet, the radiographing order information indicated by a doctor is printed. The radiographing order information includes information regarding a patient (hereinafter, it is called "patient information") such as a name of the patient to be radiographed, a sex and the like, and information regarding radiography (hereinafter, it is called "radiography information") such as a radiographing condition, a radiographing method and the like, which instructs a patient to be radiographed, a method to be used, and the like.

However, with the above-mentioned radiographing method, since the operator confirms the radiographing condition and the like with the radiographing order information printed on the order sheet, if there are a plurality of radiographing orders to one patient or a plurality of patients to be radiographed, each radiographing order information is printed on different order sheets. Therefore, it is complicated to set correspondence of a patient to radiographing order information and thereby a mistake, such as an oversight of radiographing order information, a mix-up between a patient and radiographing order information or the like may happen.

If the oversight of radiographing order information happens, the operator has to convey the set of radiographing equipments to the patient's location to perform radiography to the patient again. Therefore, it is inefficient and causes a large burden on the patient. Further, if the mix-up between a patient and radiographing order information happens, the doctor is not able to make an accurate decision based on a medical image, and therefore it decreases diagnosis precision. Consequently, handling of radiographing order information requires accuracy and safety.

### SUMMARY OF THE INVENTION

An object of the present invention is to provide a medical image terminal device, a medical image radiographing system, a display control method and program capable of performing radiography efficiently and preventing radiographing errors by setting correspondence of a patient to radiographing order information accurately and easily.

Here, of course, each section composing a medical image terminal device, such as a receiving section, a storage, an input section, a display control section or the like, may be formed by one or a plurality of units to each other, or one or a plurality of units may be a common unit for a plurality of sections among the receiving section, the storage, the input section, the display control section and the like. As an example of one or a plurality of units being a common unit for a plurality of sections among the receiving section, the storage, the input section and the display control section, a case that the input section is composed of a barcode reader with a transmitting function and a receiver, which is also the receiving section, can be cited firstly, and a case that the display control section has a CPU incorporating internal memory, which is also the storage, can be cited secondly.

In accordance with a first aspect of the present invention, a medical image terminal device comprises: a receiving section to receive identification information of a patient to be radiographed and radiographing order information including information regarding radiography; a storage section to store the radiographing order information received; an input section to input the identification information of the patient in; and a display control section to obtain the radiographing order information stored in the storage section based on the identification information of the patient input and display the radiographing order information on a display section.

In accordance with a second aspect of the present invention, a medical image radiographing system comprises an information management apparatus to manage identification information of a patient to be radiographed and radiographing order information including information regarding radiography, and a medical image terminal device connected to the information management apparatus through a network, wherein the information management apparatus comprises a transmitting section to transmit the radiographing order information, the medical image terminal device comprises: a receiving section to receive the radiographing order information; a storage section to store the radiographing order information received; an input section to input the identification information of the patient in; and a display control section to obtain the radiographing order information stored in the storage section based on the identification information of the patient input, and display the radiographing order information on a display section.

In accordance with a third aspect of the present invention, a display control method comprises: receiving identification information of a patient to be radiographed and radiographing order information including information regarding radiography; storing the radiographing order information received; obtaining the radiographing order information stored based on the identification information of the patient input; and displaying the radiographing order information obtained on a display section.

In accordance with a fourth aspect of the present invention, a program, when the program is loaded onto a medical image terminal device to control display, makes the medical image terminal device execute: receiving identification information of a patient to be radiographed and radiographing order information including information regarding radiography; storing the radiographing order information received; obtaining the radiographing order information stored based on the identification information of the patient input; and displaying the radiographing order information obtained on a display section.

According to the device of the first aspect, the system of the second aspect, the method of the third aspect or the program of the fourth aspect of the present invention, by only inputting identification information of a patient, an operator can have radiographing order information corresponding to the patient displayed on a display section. Therefore, it is possible for the operator to omit labor of, for example, setting correspondence of the radiographing order information printed on an order sheet to the identification information of the patient. As a result, it is possible to recognize the radiographing order information of the patient easily, and thereby it is possible to perform radiography efficiently. Further, it is possible to prevent a man-caused mistake such as operator's mix-up between a patient and radiographing order information.

Preferably, in the device of the first aspect of the present invention, the display control section, when there are a plurality of radiographing order information obtained based on the identification information of the patient, displays the plurality of radiographing order information as a list on the display section.

Preferably, in the system of the second aspect of the present invention, the display control section, when there are a plurality of radiographing order information obtained based on the identification information of the patient, displays the plurality of radiographing order information as a list on the display section.

Preferably, the method of the third aspect of the present invention further comprises, when there are a plurality of radiographing order information obtained based on the identification information of the patient, displaying the plurality of radiographing order information as a list.

Preferably, the program of the fourth aspect of the present invention further makes the medical image terminal device execute, when there are a plurality of radiographing order information obtained based on the identification information of the patient, displaying the plurality of radiographing order information as a list.

According to the device, the system, the method or the program, when there are a plurality of radiographing order information to one patient, the plurality of radiographing order information is displayed as a list on the display section. Therefore, it is possible for the operator to recognize the necessity of performing a plurality of radiography easily. Consequently, it is possible to prevent an oversight of radiographing order information and reduce an operation burden on the operator and a redundant burden on the patient.

Preferably, the device of the first aspect of the present invention is a carriageable portable terminal device.

Preferably, in the system of the second aspect of the present invention, the medical image terminal device is a carriageable portable terminal device.

According to the device or the system, for example, if it is necessary to perform radiography at a patient's location, by only bringing the carriageable portable terminal device, it is possible to obtain necessary radiographing order information of the patient at the patient's location.

Preferably, in the device of the first aspect of the present invention, when one among the radiographing order information displayed is chosen and cassette identification information is input, the device sets correspondence of the cassette identification information input to the radiographing order information chosen.

Preferably, in the system of the second aspect of the present invention, when one among the radiographing order information displayed is chosen and cassette identification information is input, the medical image terminal device sets correspondence of the cassette identification information input to the radiographing order information chosen.

Preferably, the method of the third aspect of the present invention further comprises, when one among the radiographing order information displayed is chosen and cassette identification information is input, setting correspondence the cassette identification information input to the radiographing order information chosen.

Preferably, the program of the fourth aspect of the present invention further makes the medical image terminal device execute, when one among the radiographing order information displayed is chosen and cassette identification information is input, setting correspondence of the cassette identification information input to the radiographing order information chosen.

### BRIEF DESCRIPTION OF THE DRAWINGS

The present invention will become more fully understood from the detailed description given hereinafter and the accompanying drawing given by way of illustration only, and thus are not intended as a definition of the limits of the present invention, and wherein:
FIG. 1 is a conceptual view showing a system structure of a medical image radiographing system 1 according to an embodiment to which the present invention is applied,
FIG. 2 is a block diagram showing a functional structure of a portable-type radiographing information device 10 shown in FIG. 1,
FIG. 3 is a view showing a data structure example of a radiographing order information file 161 stored in a storage 16 shown in FIG. 2,
FIG. 4 is a block diagram showing a functional structure of an information management apparatus 20 shown in FIG. 1,
FIG. 5A is a flow chart illustrating a radiographing order information obtaining process executed by a CPU 11 shown in FIG. 2, FIG. 5B is a flow chart illustrating a radiographing order information transmitting process executed by a CPU 21 shown in FIG. 4,
FIG. 6 is a flow chart illustrating a radiographing order information searching process executed by the CPU 11 shown in FIG. 2,
FIG 7A is a view showing an example of representation displayed on a display section 13 of the portable-type radiographing information device 10, and FIG. 7B is a view showing another example of representation displayed on the display section 13 of the portable-type radiographing information device 10.

### EMBODIMENTS OF THE INVENTION

Hereinafter, an embodiment of the present invention will be explained in detail with reference to figures. However, the range of the invention is not limited to illustrated examples. By the way, hereafter, a medical image radiographing apparatus wherein a medical image terminal device characteristic to the present invention is a portable-type radiographing information device (hereinafter, it is called "PDA (Personal Digital Assistant)"), which is carriageable, a medical image radiographing apparatus is a movable radiographing apparatus, and radiography is performed at a patient's location, will be explained as an example.

First, a structure of the present embodiment will be explained.

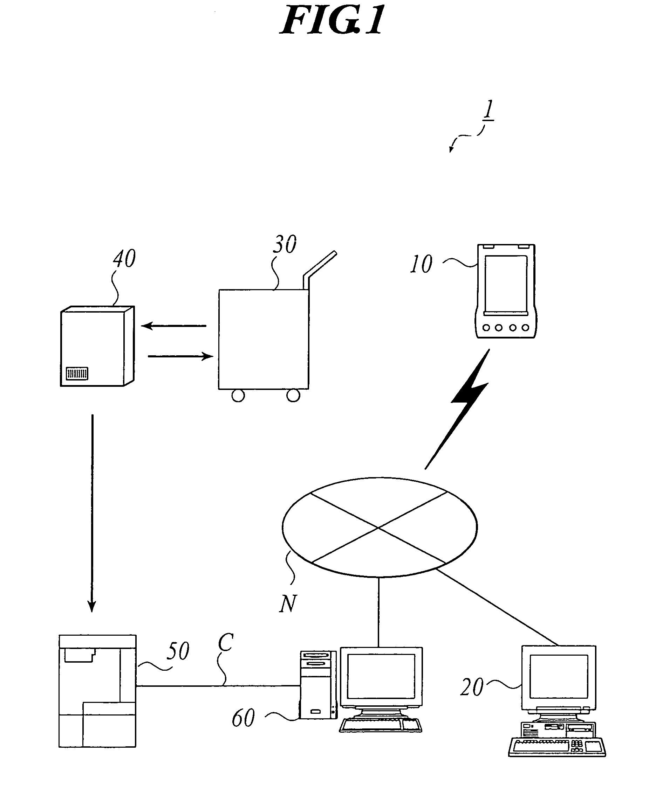

FIG. 1 is a conceptual view showing a system structure of a medical image radiographing system 1 according to the present invention. As shown in FIG. 1, the medical image radiographing system comprises a PDA 10, an information management apparatus 20, a medical image radiographing apparatus 30, a cassette 40, a medical image reading apparatus for cassette use 50, a control apparatus 60 and the like. The PDA 10, the information management apparatus 20 and the control apparatus 60 are connected to a network N and capable of accessing each other.

The PDA 10, a portable-type radiography information device carried by an operator operating the medical image radiographing apparatus 30, receives radiographing order information from the information management apparatus 20 through the network N to store it, and searches radiographing order information corresponding to a patient ID among the stored radiographing order information to display it.

The information management apparatus 20, a terminal to integrally manage radiographing order information instructed by a doctor, extracts radiographing order information according to request instruction from the PDA 10 and transmits the extracted radiographing order information to the PDA 10. Here, as another information management apparatus, an entry terminal (not shown) to enter reservation request of radiographing order information may be applicable, or an information management system such as an HIS (Hospital Information System), an RIS (Radiology Information System) or the like may be applicable as well.

The medical image radiographing apparatus 30, a movable medical image radiographing apparatus, performs radiography to a patient at a patient's location and records a medical image in the cassette 40, which is detachable to the medical image radiographing apparatus 30. The cassette 40, incorporating a photostimulable phosphor sheet therein to accumulate a part of radiation energy, accumulates a part of energy irradiated from a radiation source which has transmitted through a subject located between the radiation source and the cassette, in the photostimulable phosphor sheet.

The medical image reading apparatus 50, a medical image reading apparatus to read the medical image recorded in the cassette 40, irradiates excitation light to the photostimulable phosphor sheet of the cassette 40, photoelectrically converts fluorescence emitted from the sheet into an image signal, and A/D converts the obtained image signal into a medical image.

The control apparatus 60, an apparatus to control reading operation of the medical image reading apparatus 50, sets correspondence of radiographing order information received from the information management apparatus 20 to the medical image read out of the cassette 40 to manage the medical image.

The network N can take various line forms such as LAN (Local Area Network), WAN (Wide Area Network), Internet or the like. Here, provided that a medical institution such as a hospital or the like permits, the network may be radio communication or infrared-ray communication. Since the radiographing order information includes important patient information, while transmitted and received, preferably the radiographing order information is encoded.

Next, each apparatus of the system, which is a main component of the present invention, will be explained in detail.

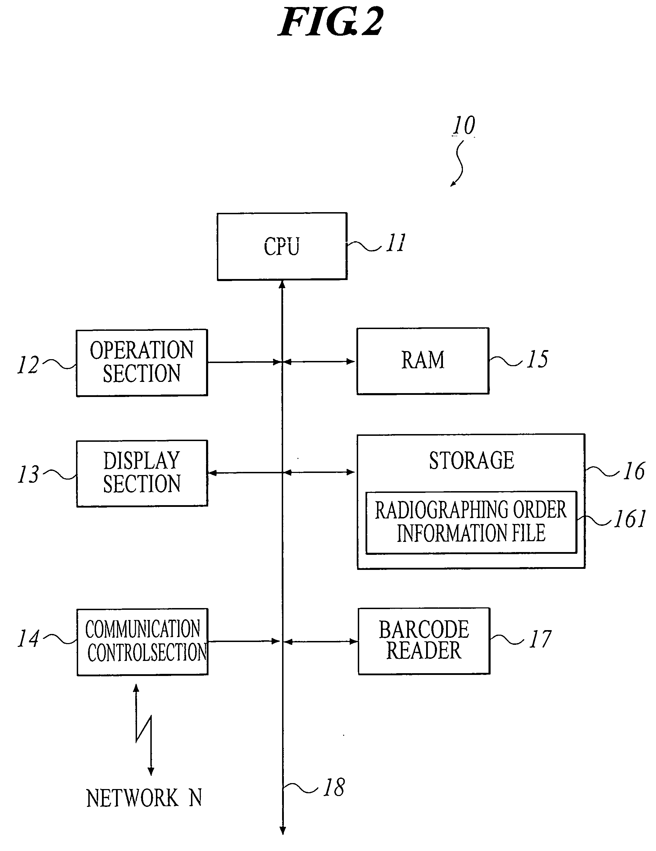

FIG. 2 is a block diagram showing a functional structure of the PDA 10. As shown in FIG. 2, the PDA 10 comprises a CPU 11, an operation section 12, a display section 13, a communication control section 14, a RAM 15, a storage 16, a barcode reader 17 and the like. Each section is connected to each other via a bus 18.

The CPU (Central Processing Unit) 11 develops a system program and a program assigned among various types of application programs stored in the storage 16 into the RAM 15 to control each section of the PDA 10 integrally according to the programs.

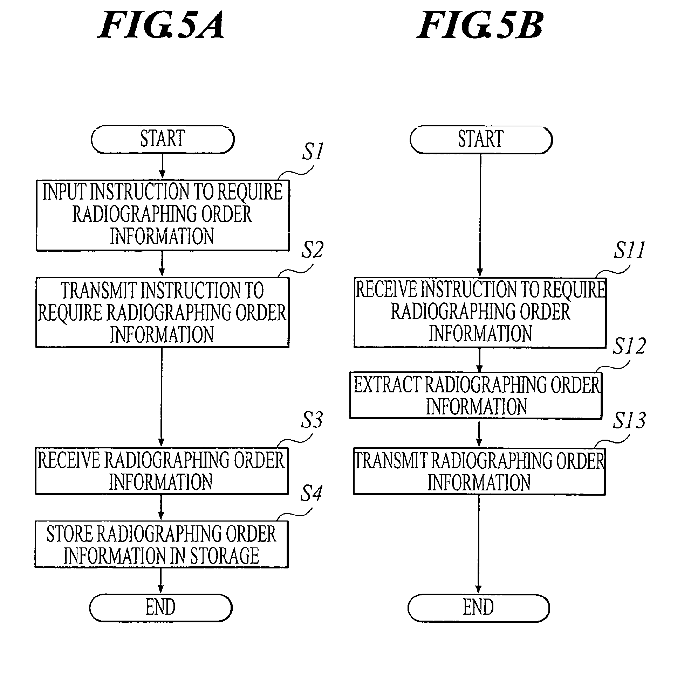

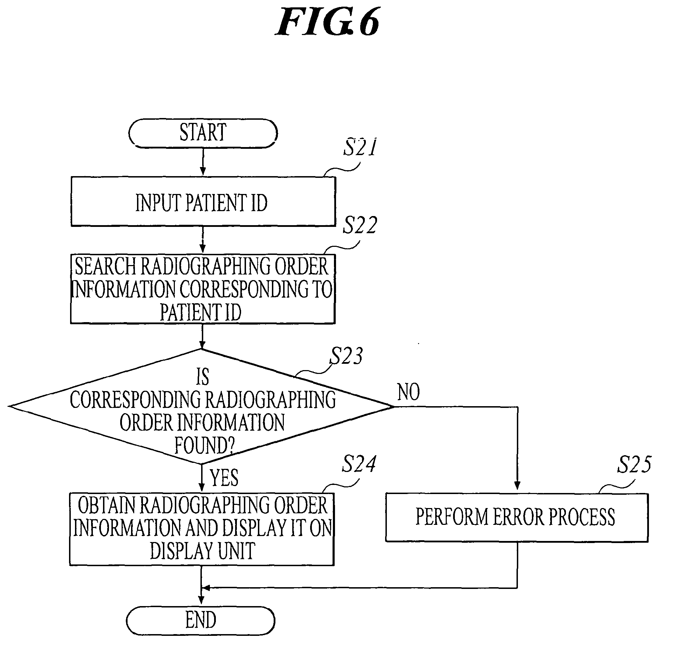

Concretely, the CPU 11 downloads a radiographing order information obtaining process program and a radiographing order information searching program from the storage 16 to execute a radiographing order information obtaining process (see FIG. 5A and 5B) and a radiographing order information searching process (see FIG. 6), which will be explained later.

When executing the radiographing order information obtaining process, in response to operator's input of request instruction of radiographing order information at the operation section 12, the CPU 11 controls the communication control section 14 to connect to the network N and transmits the input request instruction to the information management apparatus 20. Further, the CPU 11 controls the communication control section 14 to receive the radiographing order information from the information management apparatus 20, and stores the received radiographing order information in a radiographing order information file 161 of the storage 16, which will be explained later.

Further, when executing the radiographing order information searching process, in response to operator's input of a patient ID, which is identification information of the patient, at either the operation section 12 or the barcode reader 17, the CPU 11 searches radiographing order information corresponding to the input patient ID in the radiographing order information file 161. If the radiographing order information file 161 stores radiographing order information corresponding to the input patient ID, the CPU 11 obtains the corresponding radiographing order information to display it on the display section 13. Here, if the radiographing order information file 161 stores a plurality of radiographing order information corresponding to the input patient ID, the CPU 11 obtains the plurality of radiographing order information to display them on the display section 13. Further, if the radiographing order information file 161 does not store radiographing order information corresponding to the input patient ID, the CPU 11 executes an error process and displays an error screen on the display section 13.

The operation section 12, composed of cursor keys, numeric keys, various types of function keys and the like, outputs a operation signal corresponding to a key pushed by the operator to the CPU 11. For example, the operation section 12 accepts input of the patient ID, which is identification information of the patient, according to operation by the operator. Here, the operation section 12 may comprise a pointing device such as a touch panel or the like, or other types of input device according to need.

The display section 13, a display means comprising a display such as an LCD (Liquid Crystal Display) or the like, displays various information such as the radiographing order information, the patient ID and the like based on display instruction from the CPU 11.

The communication control section 14, a receiving section to receive the radiographing order information, achieves a receiving means and a transmitting means with a network interface card, a modem, a terminal adaptor or the like and controls communication with external devices connected to the network N. For example, the communication control section 14, while establishing radio communication with the information management apparatus 20, transmits the request instruction of the radiographing order information and receives the radiographing order information from the information management apparatus 20 through the network N. By the way, in this case, radio communication may be established by means of a cell-phone such as a PHS or the like.

The RAM (Random Access Memory) 15 forms a work memory area to store the above-mentioned assigned application programs, input instruction, input data, processing results and the like.

The storage 16 comprises a storage medium (not shown) in which programs and data are stored in advance. The storage medium stores a system program, various types of application programs corresponding to the system program, data processed by the various types of application programs. Further, the storage medium is composed of a magnetic storage medium, an optical storage medium or a semiconductor memory, and attached with the storage 16 either fixedly or detachably.

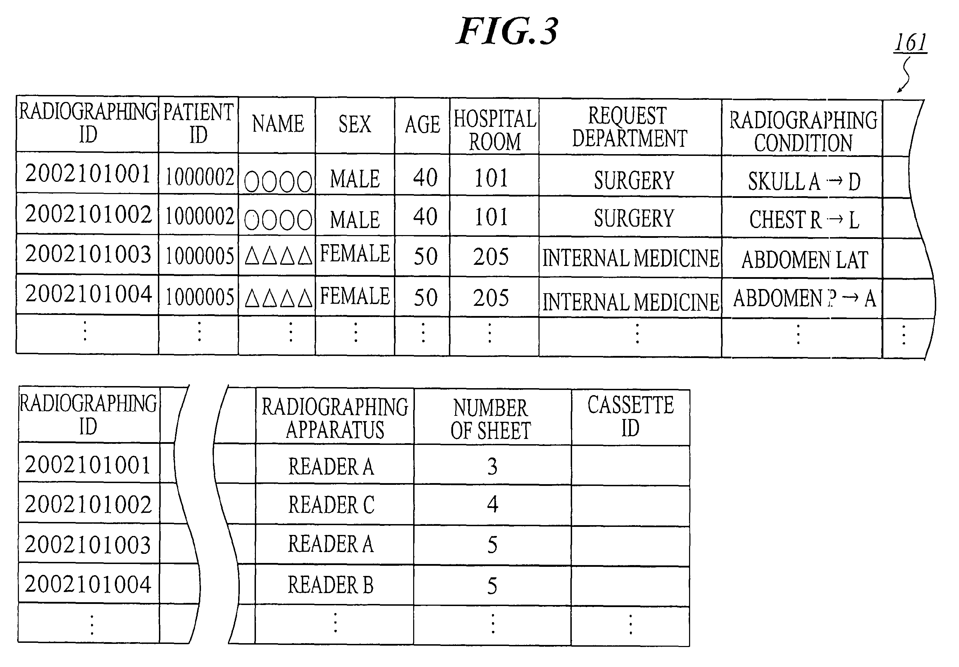

Further, the storage 16 is a storing section to store the radiographing order information, and has the radiographing order information file 161 to store the radiographing order information received from the information management apparatus 20. With reference to FIG. 3, the radiographing order information file 161 will be explained. FIG. 3 is a view showing a data structure example of the radiographing order information file 161. As shown in FIG. 3, the radiographing order information file 161 has items to store a radiographing order ID, a patient ID, a name, a sex, an age, a hospital room, a request department, a radiographing condition including a radiographic part, a radiographing direction and the like, a radiographing apparatus, the number of sheet and a cassette ID, and stores data corresponding to each of the items for each radiographing order information.

In the item of the radiographing order ID, an identification code uniquely distributed to identify radiography (for example, 20020101001, 20020101002, 20020101003, ...) is stored. In the item of the patient ID, an identification code uniquely distributed to identify a patient for the radiography (for example, 1000002, 100005, ...) is stored. In the item of the name, letter information indicating a name of the patient to be radiographed is stored, and in the item of the sex, letter information indicating a sex of the patient to be radiographed is stored. In the item of the age, numerical information indicating an age of the patient to be radiographed is stored, and in the item of the hospital room, letter information indicating a hospital room to be used for the radiography is stored.

In the item of the request department, letter information indicating a department requesting the radiography is stored, and in the item of the radiographing condition, information indicating a radiographic part and a radiographing direction (for example, SKULL A→P, SKULL P→ A, CHEST P→A, ...) is stored. In the item of the radiographing apparatus, letter information indicating a type of the radiographing apparatus to be used for the radiography is stored, and in the item of the number of sheet, the number of sheet to be used for the radiography is stored as numerical information. In the item of the cassette ID, a cassette ID (for example, information read out of a barcode or the like) uniquely distributed to identify a cassette used for the radiography is stored. However, data of the cassette ID is not stored until the radiography. The storage 16 stores the cassette ID input by the operator at the time of the radiography.

Further, in the radiographing order information, the patient information may include, in addition to the patient ID, the name, the sex and the age, various types of information, for example, a doctor in charge, warning information to warn an infectious disease or the like, existence of drug allergy, a pregnancy status, an additional medical history, special care necessity such as a wheelchair, a stretcher or the like, a clinical diagnosis name, a secret matter and the like. Further, the radiography information may include, in addition to the radiographing condition including the radiographic part, the radiographing direction and the like, the radiographing apparatus and the number of sheet, various types of information, for example, a radiographing method (simple radiography, contrast radiography or the like), a date to radiograph and the like.

The barcode reader 17, an input section to input the patient ID in, has a scanner, which is an optical reading device. The barcode reader 17 obtains information indicated by a barcode by reading the barcode with the scanner and decoding the read information with a predetermined standard. For example, the barcode reader 17 reads a barcode attached with a bedside of the patient or a part of the patient body to obtain the patient ID. Further, at the time of radiography, the barcode reader 17 obtains the cassette ID from a barcode attached with the cassette to be used to record a medical image. Here, the predetermined standard can be, JAN code, UPC code, CODE 39, CDE 93, CODE 128, NW-7, INDUSTRIAL 2 of 5, ITF code or the like.

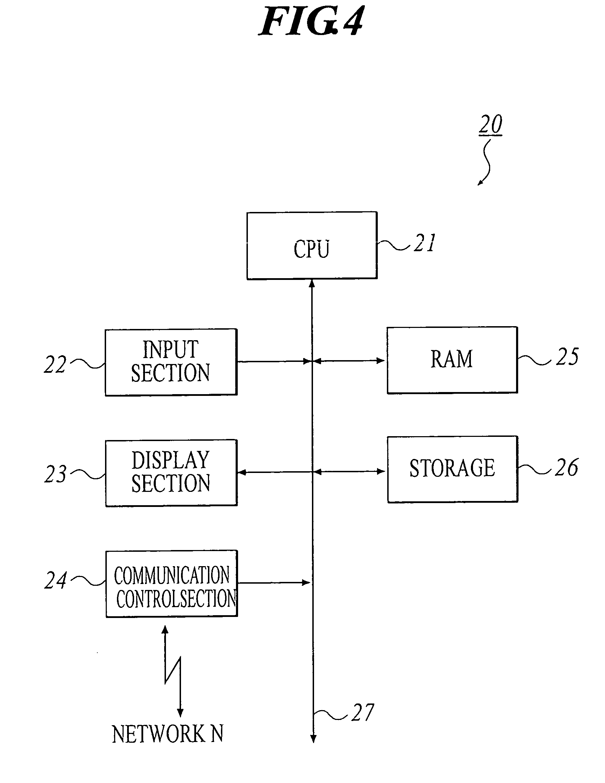

FIG. 4 is a block diagram showing a functional structure of the information management apparatus 20. As shown in FIG. 4, the information management apparatus 20 comprises a CPU 21, an input section 22, a display section 23, a communication control section 24, a RAM 25, a storage 26 and the like. Each section is connected to each other via a bus 27.

The CPU 21 downloads a system program and various types of control programs stored in the storage 26, develops them into the RAM 25 and integrally controls operation of each section according to the control programs. Further, the CPU 21 executes various processes according to the programs developed into the RAM 25, and temporarily stores results of the processes in the RAM 25 as well as displays them on the display section 23.

Concretely, the CPU 21 downloads a radiographing order information transmitting process program from the storage 26 and executes a radiographing order information transmitting process (see FIG. 6), which will be explained later. When executing the radiographing order information transmitting process, in response to receiving request instruction of the radiographing order information from the PDA 10 via the network N, for example, the CPU 21 extracts the radiographing order information from a radiographing order information DB (not shown), which integrally manages the radiographing order information in the hospital, and transmits the extracted radiographing order information to the corresponding PDA 10. Here, for example, the extraction of the radiographing order information may be made with an arbitrary selection of the operator who operates the information management apparatus 20, or when the radiographing order information is distributed to the operator or the PDA 10 in advance, the radiographing order information corresponding to the identification information unique to the operator or the PDA 10, which is transmitted along with the request information, may be extracted.

The input section 22, including a keyboard with cursor keys, numeric keys, various function keys, outputs a pushed signal corresponding to a key pushed on the keyboard to the CPU 21. Here, the input section 22 may comprise a pointing device such as a mouse, a touch panel or the like, or another inputting device.

The display section 23, composed of an LCD, a CRT (Cathode Ray Tube) or the like, displays input instruction, data and the like from the input section 22 according to instruction of display signals input from the CPU 21.

The communication control section 24, composed of a LAN adapter, a TA (Terminal Adapter) or the like, controls communication of each device connected to the network N via a communication line such as a lease line, an ISDN line or the like.

The RAM 25 forms a memory area to temporarily store a system program, which is executable by the CPU 11 and downloaded from the storage 26, a control program, input and output data, parameters and the like, in various processes executed and controlled by the CPU 21,

The storage 26, composed of a HDD (Hard Disc Drive), an nonvolatile semiconductor memory or the like, stores the system program executed by the CPU 21, various types of process programs corresponding to the system program, results of the processes and the like. Further, the storage 26 comprises a memory medium (not shown) in which the programs and data are stored in advance. The memory medium is composed of a magnetic memory medium, an optical memory medium or a semiconductor memory, and attached with the storage 26 either fixedly or detachably. These various types of programs are stored as downloadable program codes, and the CPU 21 executes operation according to the program codes.

Next, operation of the present embodiment will be explained.

Here, a program to achieve each function written in flow charts which will be explained later, is stored in either the storage 16 of the PDA 10 or the storage 26 of the information management apparatus 20 as a program code downloadable for the computer. Either the CPU 11 of the PDA 10 or the CPU 21 of the information management apparatus 20 sequentially executes operation according to the program code.

FIG. 5A is a flow chart illustrating the radiographing order information obtaining process executed by the CPU 11 of the PDA 10. As shown in FIG. 5A, when the operator inputs request instruction of the radiographing order information at the operation section 12 (Step S1), the CPU 11 controls the communication control section 14 to transmit the request instruction to the information management apparatus 20 (Step S2).

Next, when the PDA 10 receives the radiographing order information from the information management apparatus 20 (Step S3), the PDA 10 stores the received radiographing order information in the radiographing order information file 161 of the storage 16 (Step S4), and the radiographing order information obtaining process is completed.

FIG. 5B is a flow chart illustrating the radiographing order information transmitting process executed by the CPU 21 of the information management apparatus 20. As shown in FIG. 5B, when the information management apparatus 20 receives the request instruction of the radiographing order information from the PDA 10 through the network N (Step S11), the CPU 21 extracts the radiographing order information from the storage 26 (Step S12). Then, the CPU 21 transmits the extracted radiographing order information to the corresponding PDA 10 (Step S13) and the radiographing order information transmitting process is completed.

FIG. 6 is a flow chart illustrating the radiographing order information searching process executed by the CPU 11 of the PDA 10. As shown in FIG. 6, when the patient ID is input to the PDA 10 through the barcode reader 17 (Step S21), the CPU 11 obtains the radiographing order information file 161 stored in the storage 16 to search the radiographing order information corresponding to the input patient ID within (Step S22).

Here, if the radiographing order information file 161 has the radiographing order information corresponding to the patient ID (Step S23; YES), the CPU 11 obtains the corresponding radiographing order information and displays it on the display section 13 (Step S24) and the radiographing order information searching process is completed. On the other hand, if the radiographing order information file 161 does not have the radiographing order information corresponding to the patient ID (Step S23; NO), the CPU 11 executes an error process to display an error screen indicating that there is no corresponding radiographing order information (Step S25) and the radiographing order information searching process is completed.

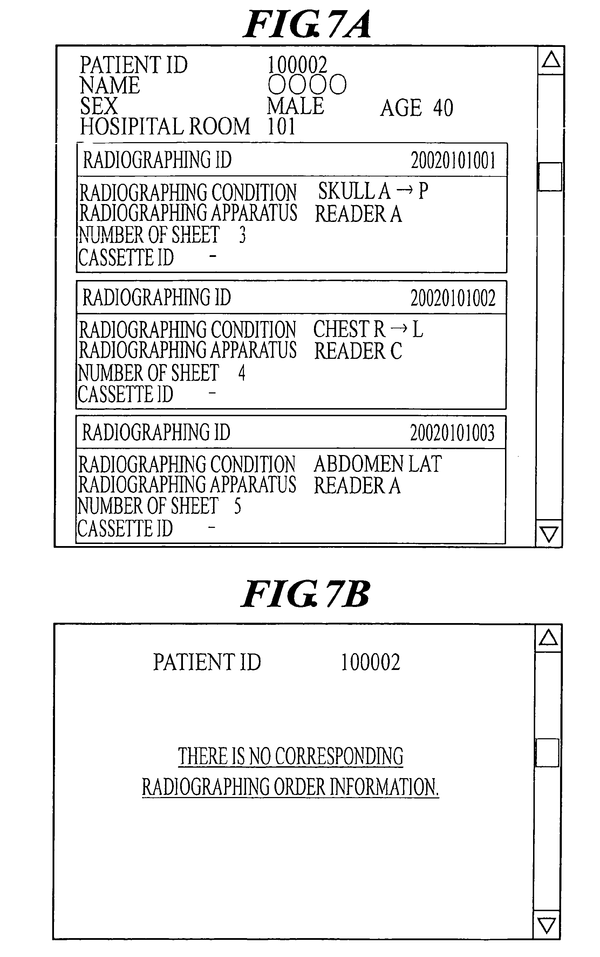

Next, with reference to FIG. 7A and FIG. 7B, the display screen displayed on the display section 13 in the above-mentioned radiographing order information searching process will be explained. FIG. 7A is a view showing an example of the display screen displayed in Step S24 of FIG. 6. As shown in FIG. 7A, in the upper part of the display screen, the patient information corresponding to the patient ID input by the operator is displayed and it is possible to see the patient ID, the name, the sex, the age, the hospital room and the like visually.

Further, below the patient information, a plurality of radiographing order information is displayed as a list, and the radiographing condition, the radiographing apparatus, the number of sheet, the cassette ID for each radiographing ID are shown. Here, when the operator inputs the cassette ID of the cassette 40 through the barcode reader, the input data is displayed in the item of the cassette ID. In other words, when the operator inputs the cassette ID of the cassette 40, in the item of a cassette ID of a record selected among the displayed records (in order words, a record getting focus by the operator's operation), the cassette ID is input. Then, data of the input cassette ID is displayed.

Further, FIG. 7B is another example of the display screen displayed in Step S25 of FIG. 6. As shown in FIG. 7B, if the radiographing order information file 161 does not have radiographing order information corresponding to the input patient ID, a message indicating that there is no corresponding radiographing information is displayed. Therefore, the operator can avoid the mix-up of a patient to be radiographed and an input mistake of the patient ID from happening.

As mentioned, according to the present embodiment, the PDA 10, in advance, obtains the radiographing order information from the information management apparatus 20 and stores it in the radiographing order information file 161 of the storage 16. Then, at the time of radiography, when the patient ID is input, the PDA 10 obtains the radiographing order information corresponding to the patient ID from the radiographing order information file 161 and displays it on the display section 13.

Therefore, since it is possible to obtain necessary radiographing order information quickly at a patient's location by only bringing the PDA 10, which is easy to carry, at the time of radiography, it is not necessary to spend extra time to print the radiographing order information on the order sheet in advance, or bring a plurality of order sheets as required documents. Consequently, it is possible to avoid being incapable of radiography due to the misplacement of the order sheet and thereby it is possible to perform radiography efficiently.

Further, at the time of radiography, when the operator inputs the patient ID of the patient to be radiographed to the PDA 10, the corresponding radiographing order information is displayed as a list on the display section 13 of the PDA 10. Therefore, it is easy to handle the radiographing order information and thereby it is possible to avoid the mix-up between the patient and the radiographing order information. Further, even when a plurality of radiographing orders are issued to one patient, it is possible to avoid an oversight of the radiographing order information. Consequently, it is possible to perform a plurality of radiography to one patient efficiently, and thereby it is possible to reduce a burden on the patient.

Further, when the operator inputs the patient ID to the PDA 10, it is possible to choose whether to input it manually at the operation section 12 or to have the barcode reader 17 read the barcode. As a result, it is possible to choose a inputting method depending on a patient condition or an operation circumstance, and thereby it is convenient. Further, by using the barcode reader 17 to input the patient ID, it is possible to avoid an input mistake, and thereby it is possible to set correspondence of the patient to the radiographing order information accurately.

Here, the above-mentioned description of the present embodiment is an example of a medical image terminal device and a medical image radiographing system suitable for the present invention, and therefore the present invention is not limited to the description.

For example, in the present embodiment, a case where a medical image terminal device is applied to a portable-type PDA 10 and a medical image radiographing apparatus 30 is applied to a movable radiographing apparatus to perform radiography at a patient's location, is explained as an example. However, for example, the medical image terminal device may be composed of a general personal computer (PC) or the like, and the medical image radiographing apparatus 30 may be a radiographing apparatus fixedly installed in a radiography room. Further, the PDA 10 may be a portable communication terminal, for example, a cell-phone, a laptop PC or the like.

Further, the illustration of the patient information and the radiographing information included in the radiographing order information is an example, and therefore they may include other types of information according to need. For example, as the identification information of the patient, a structure where the patient ID which is a combination of a plurality of numerical values or its barcode, is applied, is explained as an example. However, it is not limited to the structure. As method to identify a patient, a patient name, a date of birth, a fingerprint, a palm print, a voice print, a face, an iris, an ID card or the like, may be also applicable. In this case, preferably the PDA 10 comprises a input section with which it is possible to input each of the data.

Further, a structure where a patient is identified with a combination of a plurality of the above-mentioned information, may be applicable. This enables to set correspondence of the patient to the radiographing order information more accurately.

In addition, the structure detail and the operation detail of each component of the medical image radiographing system 1 can be suitably modified without departing from the essence of the present invention.

As described, the example that the cassette contains a phosphor plate wherein a photostimulable phosphor layer is formed on a support member, and the medical image reading apparatus is a CR apparatus, has been explained. However, the range of the present invention is not limited to the example above. As another example, a combination of a cassette incorporating a Flat Panel Detector (FPD) and an information management apparatus receiving a medical image from the cassette, may be applicable.

The entire disclosure of Japanese Patent Application No. Tokugan 2002-317226 filed on October 31, 2002 including a specification, claims, drawings and summaries are incorporated herein by reference in their entirety.

## Claims

1. A medical image terminal device comprising:
a receiving section to receive identification information of a patient to be radiographed and radiographing order information including information regarding radiography;
a storage section to store the radiographing order information received;
an input section to input the identification information of the patient in; and
a display control section to obtain the radiographing order information stored in the storage section based on the identification information of the patient input and display the radiographing order information on a display section.

2. The device of claim 1, wherein the display control section, when there are a plurality of radiographing order information obtained based on the identification information of the patient, displays the plurality of radiographing order information as a list on the display section.

3. The device of any one of claims 1 and 2, wherein the device is a carriageable portable terminal device.

4. The device of any one of claims 1 to 3, wherein when one among the radiographing order information displayed is chosen and cassette identification information is input, the device sets correspondence of the cassette identification information input to the radiographing order information chosen.

5. A medical image radiographing system comprising an information management apparatus to manage identification information of a patient to be radiographed and radiographing order information including information regarding radiography, and a medical image terminal device connected to the information management apparatus through a network,
wherein the information management apparatus comprises a transmitting section to transmit the radiographing order information,
the medical image terminal device comprises:
a receiving section to receive the radiographing order information;
a storage section to store the radiographing order information received;
an input section to input the identification information of the patient in; and
a display control section to obtain the radiographing order information stored in the storage section based on the identification information of the patient input, and display the radiographing order information on a display section.

6. The system of claim 5, wherein the display control section, when there are a plurality of radiographing order information obtained based on the identification information of the patient, displays the plurality of radiographing order information as a list on the display section.

7. The system of any one of claims 5 and 6, wherein the medical image terminal device is a carriageable portable terminal device.

8. The system of any one of claims 5 to 7, wherein when one among the radiographing order information displayed is chosen and cassette identification information is input, the medical image terminal device sets correspondence of the cassette identification information input to the radiographing order information chosen.

9. A display control method comprising:
receiving identification information of a patient to be radiographed and radiographing order information including information regarding radiography;
storing the radiographing order information received;
obtaining the radiographing order information stored based on the identification information of the patient input; and
displaying the radiographing order information obtained on a display section.

10. The method of claim 9, further comprising, when there are a plurality of radiographing order information obtained based on the identification information of the patient, displaying the plurality of radiographing order information as a list.

11. The method of any one of claims 9 and 10, further comprising, when one among the radiographing order information displayed is chosen and cassette identification information is input, setting correspondence the cassette identification information input to the radiographing order information chosen.

12. A program, when the program is loaded onto a medical image terminal device to control display, the program making the medical image terminal device execute:
receiving identification information of a patient to be radiographed and radiographing order information including information regarding radiography;
storing the radiographing order information received;
obtaining the radiographing order information stored based on the identification information of the patient input; and
displaying the radiographing order information obtained on a display section.

13. The program of claim 12, further making the medical image terminal device execute, when there are a plurality of radiographing order information obtained based on the identification information of the patient, displaying the plurality of radiographing order information as a list.

14. The method of any one of claims 12 and 13, further making the medical image terminal device execute, when one among the radiographing order information displayed is chosen and cassette identification information is input, setting correspondence of the cassette identification information input to the radiographing order information chosen.
